# EUROPEAN PATENT APPLICATION

(11) **EP 0 963 758 A2**
(43) Date of publication of application: **15.12.1999**
(21) Application number: 98201566.1
(22) Date of filing: 12.05.1998
(51) Int. Cl.: A61K 47/48

(54) **Synthetic polyaminoacid complexes for delivery of nucleic acids to target cells**

(30) Priority: 07.05.1998 EP 98201454
(71) Applicant: UNIVERSITEIT GENT, B-9000 Gent (BE)
(72) Inventor: Schacht, Honoré, 8640 Staden (BE)
(74) Representative: Bird, William Edward

(57) **Abstract**

A synthetic polymer-based carrier vehicle and a method of constructing the synthetic polymer-based carrier vehicle for delivery of nucleic acid material to target cells in biological systems are described. The method comprising the steps of:
a) providing a cationic polyelectrolyte polymer material including a
   poly 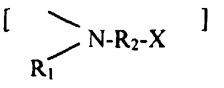 derivative of an amino acid or a synthetic amino acid; and
b) bringing the nucleic acid material into association with said cationic polyelectrolyte polymer material to form by self-assembly therebetween a polyelectrolyte complex which provides a nucleic acid containing cationic polymer core for said carrier vehicle.

The synthetic polymer-based carrier vehicle includes the amino acid derivative made by the method. The derivative is preferably a polyglutamine or polyasparagine derivative.

## Description

The present invention relates to the delivery of nucleic acid material to target cells in biological systems and to the construction of delivery vehicles for this purpose, especially in connection with gene therapy. The present invention may also relate to a polymer system which may be condensed with nucleic acids to form part of a delivery system for the nucleic acids to target cells in biological systems.

### BACKGROUND

The possibility of delivering genes into somatic cells raises many promising new therapeutic opportunities, although the difficulty of efficient delivery to target cells *in vivo* currently represents a major barrier to progress. Despite the range of techniques available for *in vitro* transfection of cells, many of these techniques e.g. calcium phosphate precipitation, electro-permeabilisation, etc., cannot be applied *in vivo*. Most animal and clinical studies have relied on the use of liposomal or viral vectors. Cationic liposomes have shown some success *in vivo*, particularly via non-systemic routes, but they are poorly defined and their net charge is thought to inhibit effective systemic delivery because it promotes binding to plasma proteins and to extracellular matrix. At present viruses provide the most popular vectors for *in vivo* delivery, particularly with improved DNA packaging techniques. However, their inherent immunogenicity, possibility of fixing complement, poor target selectivity and difficulty of scale-up production, together with concerns over potential toxicity, seem likely to prevent their widespread acceptance and licensing. There is therefore a clear need for alternative safe and efficient DNA or gene delivery systems preferably based on fully synthetic carrier vehicles.

A synthetic carrier vehicle or vector suitable for efficient targeted delivery of DNA or other nucleic acid material *in vivo* must fulfil various biological requirements. Ideally it would be stable in the blood circulation, non-immunogenic and resistant to enzymatic degradation, capable of efficient target-discrimination, and able to penetrate the target cell membrane selectively to gain access to the nucleus, release the nucleic acid and enable efficient transcription within the target cell. For successful and versatile *in vivo* application it is very important that nucleic acid delivery vehicles should be small enough to gain access to target cells. Access to target cells frequently involves extravasation through endothelial layers, but even the hyper-permeable endothelia associated with tumours have a size restriction of about 70 nm. In addition, most forms of triggered membrane penetration act via the endosomal membrane following endocytosis and endocytic internalisation is usually limited to materials of less than 100 nm diameter. Given the large size of DNA expression vectors in free solution (typical diameter is 200 nm) it is clearly necessary for the DNA to be compressed and packaged during self-assembly with cationic polymers if the polyelectrolyte complexes thereby formed are to provide satisfactory DNA carriers and delivery vehicles.

One approach to the development of synthetic vectors or carrier vehicles for delivery of DNA has been proposed based on soluble cationic polymers designed to self-assemble with DNA of expression vectors, it having been shown previously that DNA can be condensed into polyelectrolyte complexes by the addition of polycations, rendering it easier to package. For example, simple mixing of DNA with poly(L)lysine results in formation of discrete polyelectrolyte particles whose size and capacity for spontaneous transfection can be influenced by the molecular weight of the poly(L)lysine used. Specific cell targeting groups, e.g. transferrin and/or membrane-permeabilising groups such as membrane disrupting oligopeptides, can be incorporated into such structures and significantly enhance the transfection rates achieved.

These simple polyelectrolyte DNA complexes are of limited usefulness for systemic administration due to rapid clearance following intravenous (i.v.) injection although the exact reasons for this rapid clearance are not fully understood. It does appear that these simple DNA, cationic polymer complexes are subject to destabilisation by serum proteins, especially albumin at physiological concentrations, and may be subject to degradation by serum nucleases, despite their relative stability when compared with free DNA.

Improved polyelectrolyte DNA complexes are described in co-pending, non-prepublished International patent application PCT/GB97/02965 in which preparation of synthetic polyelectrolyte vectors or nucleic acid carrier vehicles are described involving formation of a complex having a nucleic acid-containing core portion by self-assembly between cationic polymer material and nucleic acid material, especially DNA contained in an expression vector, the core portion being provided, directly or indirectly, with various other functional molecules or molecular entities including molecules of hydrophilic polymer material that provide a coating and steric shield for the core portion, thereby improving stability and biocompatibiliy of the polyelectrolyte complex. The cationic polymer material may also include as copolymer component polyamine molecules such as poly(L)lysine or poly(L)ornithine. Although the presence of a self-assembled coating of synthetic hydrophilic polymer can confer up to 100-fold stabilisation of simple DNA/cationic polymer complexes in the presence of serum proteins, at physiological concentrations of albumin optimal complexes using linear block copolymers having a cationic polymer block and a hydrophilic polymer block are often still destabilised quite quickly.

One object of the present invention is to provide improved synthetic polymer-based polyelectrolyte vectors to serve as carrier vehicles for efficient and effective delivery of nucleic acid material, and transfection of target cells, especially in connection with gene therapy or even possibly in connection with development of DNA vaccines.

Another object of the present invention is to provide a synthetic polymer system for use in a nucleic acid delivery vehicle and methods of manufacture thereof, which, in combination with nucleic acids and potentially with an outer coating of hydrophilic polymer, provides improved stability and which is less subject to destabilisation by serum proteins, especially albumin at physiological concentrations, and/or to degradation by serum nucleases.

Yet another object of the present invention is to provide a synthetic polymer system for use in a nucleic acid delivery vehicle and methods of manufacture thereof, with improved expression of genes in targeted cells, especially in connection with gene therapy.

### SUMMARY OF THE INVENTION

Preparation of synthetic polyelectrolyte vectors or nucleic acid carrier vehicles in accordance with the invention will usually involve forming a complex having a nucleic acid-containing core portion by self-assembly between cationic polymer material and nucleic acid material, especially DNA contained in an expression vector. The core portion preferably includes molecules of a derivative of an amino acid, a synthetic amino acid or amino acid-like molecule. Preferably, the amino acids or synthetic amino acids belong to the acidic amino acids. Suitable natural amino acids are glutamic or aspartic acid. The core portion preferably includes a polyglutamine or polyasparagine derivative or a derivative of a polyamine of an equivalent amino acid. These compounds may be prepared by reacting the amino acid or amino acid-like molecule with compounds having the general formula: 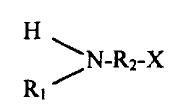 where R₁ may be hydrogen, or an alkyl, aryl, or alkylaryl group, whereby the alkyl may be methyl, ethyl, propyl, or higher orders, and the aryl group may be a phenyl, napthyl, anthryl group, or higher orders; R₂ may be any alkyl group with the general formula (CH₂)ₘ where m can be any value including 2 or greater, in particular any value between 2 and 20; and X may be an amine group. In particular X may be a group having the general formula: 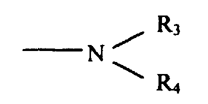 where R₃ or R₄ may be hydrogen, an alkyl group or an alkylaryl group and R₃ does not have to be the same as R₄. The alkyl group may be methyl, ethyl, propyl, or higher orders. The aryl group may be a phenyl, napthyl, anthryl group, or higher orders. Further, the X group may be a pyridino- group of the general formula: 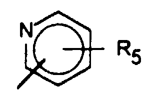 where R₅ may be hydrogen or an alkyl group such as methyl, ethyl, propyl or higher orders. The X group may also be a quinolino- group or higher order group of this type. The X group may also be an imidazolido- group of the general formula: 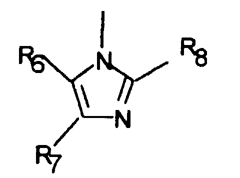 where R₆, R₇, R₈ may be hydrogen or an alkyl group such as methyl, ethyl, propyl or higher orders and R₆, R₇, R₈ do not have to be the same.

Preferably, the result of the above reaction is to produce a polymer having units with the general formula: 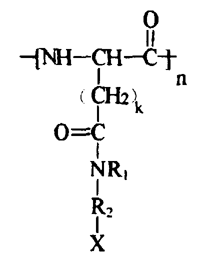 where k may be 1 or more, in particular 1 or 2, and X, R₁, R₂ are as specified above. Molecules with k = 3 or more are referred to as amino acid-like molecules or non-naturally occurring amino acids or synthetic amino acids.

The present invention also includes side groups on the cationic polymeric material described above or on the hydrophilic polymer used as a shield which may include oleyl or dioleyl groups which are expected to be membrane interacting and can influence cell up take and endosomal escape. These groups may be attached by alkylation with selected alkyl halides, e.g. bromides. The said groups may also include maleic anhydride groups which allow attachment of amines, e.g. amino polyethylene oxide, dioleyl phospatidyl ethanolamine. It is expected that the latter maleic amide acids will release the coupled amine in acid environment providing the possibility of releasing groups inside an acidic cell component, e.g. the endosomal compartment.

In particular, the core may include molecules of a poly[-N-alkyl-N-R₃, R₄] glutamine or asparagine derivative where R₃ and R₄ are as specified above. The core portion may be provided, directly or indirectly, with various other functional molecules or molecular entities including molecules of hydrophilic polymer material that provide a coating and steric shield for the core portion, thereby improving stability and biocompatibiliy of the polyelectrolyte complex. In one technique, this may be achieved by synthesising or modifying a soluble synthetic cationic polymer of the kind mentioned above so as to include or incorporate therein discrete reactive groups prior to arranging a selective self-assembly of such polymer with the nucleic acid material. The novel and inventive cationic polymers can be used to attach targeting moeities or membrane interfering moeities.

This self-assembly with the nucleic acid involves an association or binding between molecules of the polycationic component and the polyanionic nucleic acid component. In the complex so formed, the nucleic acid is condensed in the core portion and at least some of said reactive groups on the molecules of the cationic polymer component are presented at the surface thereof. These reactive groups can then be coupled or linked with hydrophilic polymer molecules and form in effect an outer shield around the polymer nucleic acid complex, thereby improving stability of the complex and presenting a hydrophilic steric barrier to interactions with cells and molecules which may be encountered in the course of *in vivo* gene therapy e.g. while circulating in the plasma after i.v. administration. The hydrophilic polymer molecules may be multivalent, i.e. will include multiple reactive groups, so that after a first reactive group binds to a reactive group of the cationic polymer and "anchors" the hydrophilic polymer other reactive groups of the latter will bind to other reactive groups of the cationic polymer, thereby cross-linking the coating or outer surface at the same time as providing a steric shielding of the nucleic acid polycation complex.

It will be understood that the term "reactive group" is used herein to denote a group that shows significant chemical reactivity, especially in relation to coupling or linking reactions with complementary reactive groups of other molecules. Also, the terms cationic and anionic denote materials which in aqueous solution at neutral pH have net positive and negative charges respectively.

Thus, as will hereinafter become apparent, the present invention may provide a cationic polymer and a nucleic acid carrier vehicle including the polymer for delivery of nucleic acid material to target cells in biological systems, e.g. *in vivo* delivery of genes or therapeutic DNA to a patient in carrying out gene therapy or DNA vaccination treatment. The cationic polymer preferably includes molecules of a derivative of an amino acid or an amino acid-like molecule as described above, in particular a derivative of polyglutamine or a derivative of a polyamine of an equivalent amino acid. A preferred example are molecules of a poly[N-alkyl-N-R₃,R₄] glutamine or asparagine derivative. The carrier vehicle may be in the form of a polyelectrolyte complex comprising a nucleic acid-containing cationic polymer core preferably associated with hydrophilic polymer material that forms an outer stabilising steric shield or coating.

The cationic polymer core will usually also be linked, directly or indirectly, to other molecular entities or moieties, especially bioactive molecules, that modify the biological and/or physico- chemical characteristics of the complex to improve suitability for use in delivering the nucleic acid material to target cells, for example, in carrying out somatic gene therapy treatment. These other molecular entities or moieties may comprise cell-receptor targeting moieties and/or other specific bioactive agents providing, for example, membrane disrupting agents, agents capable of promoting endocytic internalisation following binding to cell surface molecules, and nuclear-homing agents, useful for facilitating entry and delivery of the nucleic acid material, e.g. DNA, into cells. In particular, these other molecular species may include bioactive agents such as peptides, especially for example fusogenic amphipathic helical peptides. One particular example of the latter is peptide material known under the designation INF7 supplied by Severn Biochemicals Limited. Targeting groups as referred to above may include growth factors, antibodies or bioactive materials such as transferrin for example. The cationic polymers in accordance with the present invention may also include one or more other types of amino acids copolymerised with derivatives of L- or D- glutamic acid or L- or D- aspartic acid or other amino acids.

Nucleic acid carrier vehicles as referred to above in accordance with the invention may be constructed by means of a stepwise process in which the cationic polymer is first self-assembled with the nucleic acid material to form a complex that provides a core portion of the complete carrier vehicle. The hydrophilic polymer material may then be assembled in a subsequent step.

Thus, in one main aspect, the present invention broadly provides a method of constructing a synthetic polymer-based carrier vehicle for delivery of nucleic acid material to target cells in biological systems, said method comprising the steps of:
a) providing a cationic polyelectrolyte polymer material including molecules of a
   poly 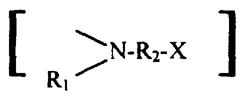 derivative of an amino acid or an amino acid-like molecule; and
b) bringing the nucleic acid material into association with said cationic polyelectrolyte polymer material to form by self-assembly therebetween a polyelectrolyte complex which provides a nucleic acid containing cationic polymer core for said carrier vehicle. R₁, R₂ and X may be as described above. Preferably, the method includes the step of reacting said polyelectrolyte complex with reactive hydrophilic polymer material so that the latter bonds to said complex and forms a hydrophilic coating that provides an outer protective steric shield and assists in stabilising the complex.

The hydrophilic polymer material of the coating associated with the nucleic acid-containing core of the complex may be bonded, at least partially, directly to the nucleic acid, for example, via reactive carboxyl groups on the polymer binding to reactive hydroxyls of alcohol groups of the nucleic acid to form ester linkages that may be broken-down subsequently by acid-catalysed or hydrophilic degradation, although it is presently preferred that the hydrophilic polymer material should be attached or linked directly only to the cationic polyelectrolyte polymer material of the nucleic acid-containing core by reactive groups which react with reactive groups of the cationic polymer material, usually, but not so necessarily, to form covalent bonds. The reactive group or groups on the cationic polymer molecules for reacting with mutually reactive groups of the hydrophilic polymer will therefore usually be selected so as to have a reactivity not present in the nucleic acid material. Also, the reactive groups carried by the cationic polymer and/or by the hydrophilic polymer may often be carried by side chains of the polymer molecules and these side chains may be pH-sensitive and acid labile, hydrolytically unstable or enzymatically biodegradable as hereinafter described.

When the nucleic acid material and the cationic polyelectrolyte polymer material self-assemble to produce the polyelectrolyte complex, reactive groups of the cationic polymer molecules will generally be exposed on the surface of the cationic polymer core, probably oriented outwardly, ready for reacting with the reactive hydrophilic polymer material in the subsequent stage. The outer protective steric shield provided by the hydrophilic coating not only assists in stabilising the complex but it can also protect the complex from unwanted biological interactions, e.g. in the course of *in vivo* gene therapy when circulating in the plasma following administration by intravenous injection.

The other molecular entities mentioned that may be carried by the cationic polymer core, e.g. cell-receptor targeting moieties and/or other specific bioactive agents, may be coupled or linked directly to cationic polymer molecules of the nucleic acid-containing polyelectrolyte complexes, either the same cationic polymer molecules as are linked to the hydrophilic polymer material or different cationic polymer molecules, again via the aforesaid reactive groups. In other embodiments, however, they may be attached to reactive groups carried by the aforesaid hydrophilic polymer material, either before or after assembly of the latter to the cationic polymer/nucleic acid complex. For this purpose, the hydrophilic polymer material may conveniently be provided by hydrophilic heterobifunctional or multifunctional polymer molecules that permit in effect the further bioactive agents to be attached to the outside of the steric shield.

In preferred embodiments the molecules of the hydrophilic coating polymer material will in fact usually be multivalent with a plurality of reactive groups so that, apart from a possible requirement to attach molecules of other bioactive agents, these reactive groups can form a plurality of cross-linkages with the nucleic acid-containing cationic polyelectrolyte polymer core of the complex which may considerably improve the stability of the construct.

The reactive groups carried by the reactive hydrophilic coating polymer may include activated esters (reactive carbonates e.g. p-nitrophenyl), thiol groups, biotin, hydrazide, anhydride or aldehyde groups which in some cases may be carried by side chains of the main polymer backbone.

Where molecules of other bioactive agents are to be attached to the hydrophilic coating polymer, it may be advantageous in some cases to provide the polymer with more than one type of reactive groups having different reactivities. This may facilitate controlling and distinguishing more readily different coupling reactions. Similarly, different kinds of reactive groups having different reactivities may be carried by the cationic polyelectrolyte polymer where different coupling reactions are required.

The reactive group or groups of the cationic polyelectrolyte polymer may be incorporated during the polymerisation or copolymerisation process a used in forming the polymer from constituent monomers. Alternatively, an existing cationic polyelectrolyte polymer already having suitable reactive groups. e.g. reactive amino groups. Or, an existing cationic polyelectrolyte polymer may be modified to introduce the required reactive groups, or to increase the reactivity of an existing reactive group, in a separate preliminary step of the process.

In one useful technique, molecules providing complementary binding groups, such as biotin molecules and avidin or streptavidin molecules, are used. Biotin molecules may be incorporated into the cationic or hydrophilic polymer material, and before use, these are linked to avidin or streptavidin molecules providing a complementary binding group attached to appropriate targeting moieties, e.g. site specific antibodies, or other bioactive agents selected by the user. Or, the reverse arrangement with the avidin or streptavidin incorporated in the cationic or hydrophilic polymer material and the biotin attached to the other molecular species may of course be used.

The invention also provides a nucleic acid carrier vehicle for *in vivo* delivery of genes or therapeutic DNA to a patient in carrying out gene therapy or DNA vaccination treatment for example, said carrier vehicle comprising a polyelectrolyte complex of the DNA or other nucleic acid material and cationic polymer material, said cationic polymer material including molecules of a derivative of an amino acid or an amino-acid-like molecule as described above, in particular derivatives of a polyglutamine or of a derivative of a polyamine of an equivalent amino acid, and as a particular example, molecules of a poly[N-alkyl-N-R₃,R₄] glutamine or asparagine derivative. One or more molecules of hydrophilic polymer material may be included in the complex to provide an outer stabilising steric shield. Other bioactive molecular species may be linked either to said cationic polymer or to said hydrophilic polymer material. The complex may be constructed in a stepwise process as hereinabove set forth.

It is anticipated that with the present invention no restriction is made on the nature of the nucleic acid material condensed with the cationic polymer. The nucleic acid material may be freely chosen and may include genomic DNA, DNA fragments of any length, plasmid DNA, cDNA, RNA, oligonucleotides, DNA expression vectors, RNA or ribozymes which is a non-limiting list. Antisense nucleic acid may sometimes also be used for certain therapies. In the DNA carrier vehicles provided by the polyelectrolyte complexes of this invention the DNA expression vector will usually be a plasmid-based expression vector incorporating an appropriate promoter sequence.

The invention also provides a method of delivering gene DNA material to a patient in carrying out somatic gene therapy treatment, said method comprising packaging the selected DNA as a expression vector in a carrier vehicle constructed as herein described, and administering the polyelectrolyte complex material forming the DNA carrier vehicle to said patient.

From another aspect the invention may be regarded as providing, for delivery of DNA to target calls in biological systems, a synthetic polymer based carrier vehicle that comprises a polyelectrolyte complex in which a DNA expression vector located in a core portion is electrostatically bound and condensed through self-assembly with a polycationic polymer including molecules of a derivative of an amino acid or an amino acid-like molecule as described above, for example, of a poly[N-alkyl-N-R₃,R₄] glutamine or asparagine derivative. After assembly with said DNA, the complex may be coupled or attached via covalent linkages to associated hydrophilic polymer material that provides a stabilising steric shield around the complex.

From yet another aspect, the invention may be regarded as consisting in a synthetic polymer-based carrier vehicle for delivery of DNA to target cells in biological systems wherein said carrier vehicle is in the form of a particle consisting of a polyelectrolyte complex comprising a DNA expression vector bound to one or more cationic polymer molecules thereby forming a DNA- containing core, the cationic polymer including molecules of a derivative of an amino acid or an amino acid-like molecule as described above, e.g. a poly[N-alkyl-N-R₃,R₄] glutamine or asparagine derivative. The core may be coupled via covalent linkages to one or more associated hydrophilic polymer molecules forming a stabilising and protective steric shield or coating around said DNA-containing complex, and one or more other molecular entities providing bioactive agents or cell receptor targeting moieties may be coupled, also via covalent linkages, to said cationic polymer material and/or to the hydrophilic polymer material, with at least some of said covalent linkages being hydrolytically unstable and/or pH sensitive or enzymatically sensitive so as to be biodegradable within the intracellular environment following endocytic uptake and internalisation by a target cell.

Alternatively, or in addition, the hydrophilic polymer may include components or linkages in its main chain backbone adapted to be biodegradable within the target cell.

Although the synthetic polymer-based nucleic acid carrier vehicles of the present invention have been designed and developed primarily for *in vivo* gene therapy, DNA vaccination can be another application and it should be appreciated that in many cases the carrier vehicles will also be suitable for *in vitro* delivery of DNA to cells, and this is also within the scope of the present invention. For example, the carrier vehicles may be used for targeted transfection of cancer cell lines and primary cells *in vitro*, and this may even be carried out on cells removed from a patient which are subsequently re-introduced as part of an *in vivo* therapeutic strategy.

In many applications of the present invention the complexes formed with the DNA will include both cationic polymer molecules coupled to a hydrophilic polymer block and cationic polymer molecules coupled to one or more targeting moieties and/or other bioactive molecules, and the stepwise method of construction of this invention may be particularly advantageous. By forming the complex of the DNA with the cationic polymer material first and then adding and incorporating the hydrophilic polymer material in a subsequent assembly step, it is believed that this procedure facilitates the production of more stable and smaller size complexes which is a most important practical feature. At physiological concentrations of albumin optimal complexes produced in accordance with the present invention have improved stability. Further, complexes constructed using the 2-stage coating or assembly procedure according to one embodiment of the present invention are likely to be even more stable since there is a reduced risk of hydrophilic polymer being trapped within the structure. It is also possible that by introducing more than one reactive group into the hydrophilic polymer, i.e. where the hydrophilic polymer is multivalent, some cross-linking will occur in the surface coating of the complex as previously mentioned.

As hereinafter described it may sometimes also be useful to use cationic polymers containing a mixture of different types of amino groups, some predominantly charged at neutral pH and others subject to significant protonation by the decreased pH during endosomal acidification by cells via endocytic processes. The endocytic vesicles have an acidic pH, typically in the range 5 to 6.5. The endocytic vesicles may fuse with the lysosomal compartment containing a variety of digestive enzymes. It is preferably if the DNA delivery vectors in accordance with the present invention do not fuse with lysosomes. Hence, a specific aspect of the present invention is the provision of DNA delivery vehicles which can escape from the endocytic vesicle without significant damage. The cationic polymers in accordance with embodiments of the present invention may contain side groups of different pKa at least some of which are not protonated at physiological pH (7.4) but become protonated at endosomal pH values. This can advantageously promote membrane rupture most likely due to osmotic and electrostatic charge effects. Moreover, the added functionalities in accordance with embodiments of the present invention such as fusogenic peptides, polyethylene oxide side chains or oleyl side chains, may promote membrane ruptures.

In some embodiments the molecular weight (weight average) of the cationic polymer in accordance with the present invention is greater than 3 kDa but below 1000 kDa, and most preferably in the range 10-200 kDa, in order to provide complexes of a suitable size. One presently favoured cationic polymer is a poly[N-alkyl-N-R₃,R₄] glutamine derivative, however, other polyaminoacids, e.g. a poly[N-alkyl-N-R₃,R₄] asparagine derivative, including copolymers with other alpha-amino acids are also included within the present invention.

The linkages coupling the molecules attached to the cationic polymer component and/or to the outer coating hydrophilic polymer component may be provided by side chains of the polymer molecules that carry the reactive groups that react to couple the molecules together. Moreover, these linkages may be either stable covalent linkages or, if it is required that the hydrophilic coating and/or attached bioactive agents be shed at the target site to permit release of the DNA, relatively unstable hydrolytic, pH-labile or enzymatically degradable linkages are appropriate. In the latter case these linkages may be susceptible to acid-catalysed cleavage, and it may be arranged so that release or conformational change of attached bioactive molecules can be triggered by the fall in pH within the endosomal or lysosomal compartment containing the complex after it has been internalised within a target cell following endocytosis.

This effect can for example facilitate the exposure and activation (or release) of membrane-active fusogenic or membrane-disrupting agents for enabling the DNA to gain access to the cytoplasm of the target cells and the exposure and activation of nuclear-homing agents, e.g. peptide nuclear-homing agents, for promoting nuclear translocation and expression. Examples of suitable pH sensitive linkages include cis-aconityl, ortho-ester, bis esters, amides or ester-amides of dimethyl maleimic acid. Alternatively, the linkages may provide a substrate designed for cleavage by cellular enzymes, e.g. they may contain peptide sequences such as GlyPheLeuGly, GlyPheAlaLeu or hydrolytically unstable groups such as esters incorporated in side chains of the polymer molecules that carry the reactive coupling groups.

It is believed that it will in fact usually be desirable to arrange for the hydrophilic polymer coating of the DNA-containing cationic polymer core to be shed or separate after internalisation in the target cell, either in the endosome or lysosome, in order to open up the structure and prepare for translocation and entry of the DNA into the nucleus. However, instead of relying upon controlled degradation of the linkages between the cationic polymer in the core and the hydrophilic polymer, it is also possible to arrange for the hydrophilic polymer to be synthesised so as to include degradable components, e.g. enzymatically degradable peptide sequences or degradable ester linkages, in the main polymer chain such that the hydrophilic polymer component would be subject to a controlled disintegration within the intracellular environment.

In general, hydrophilic polymer-shielded and/or bioactive agent modified DNA-containing polyelectrolyte complexes constructed in accordance with this invention will be particles having dimensions within a monodisperse size range of less than 100nm in diameter, generally less than 70nm in diameter and possibly approaching an ideal size of 30-40 nm diameter.

For therapeutic use these DNA or other nucleic acid carrier vehicles will usually be made up as pharmaceutical compositions formulated for administration by intravenous injection. However, intra-arterial, intraperitoneal or direct intra-tumoural injection could also be useful, and even oral administration may be feasible. Parameters which are likely to influence the performance and efficiency- of the DNA carrier vehicles of the present invention include charge ratio of the polycation to anionic nucleic acid, especially at neutral pH in aqueous solution, and also the overall hydrophilicity/hydrophobicity of the complex formed by the cationic polymer and DNA core, the molecular weight of the polymer blocks, and the overall size and conformation of the carrier vehicle.

The invention also provides new or improved methods for preparing modified cationic polymers incorporating reactive groups, and other features in connection with certain embodiments will become apparent from specific examples hereinafter described in more detail. The dependent claims define further individual embodiments of the present invention. The present invention will now be described with reference to the following drawings.

### BRIEF DESCRIPTION OF THE DRAWINGS

FIG. 1 is a composite diagram showing an example of the construction of a DNA vector or delivery vehicle with which the present invention may be used.
Fig. 2 is a schematic diagram representing the steps in the general synthesis of a cationic polymer in accordance with the present invention.
Figs. 3 to 14 are schematic diagrams representing the steps in the synthesis of cationic polymers in accordance individual embodiments of the present invention.
Fig. 15 is a diagram indicating the effect of concentration of cationic polymers in accordance with the present invention on EtBr fluorescence with DNA.
Fig. 16 is a diagram comparing the expression in transfected 293 cells of various polymer based DNA vehicles in accordance with the present invention.
Fig. 17 is a diagram indicating the stability of the complexes in accordance with the present invention with respect to albumin.

### DESCRIPTION OF THE ILLUSTRATIVE EMBODIMENTS

The present invention will be described with reference to specific embodiments and with reference to certain drawings but the invention is not limited thereto but only be the claims. For instance, the present invention will mainly be described with reference to derivatives of polyglutamines in particular derivatives of a poly[-N-alkyl-N-R',R] glutamine, however, the invention is not limited thereto but only by the claims. The present invention may also include derivatives of polyamines of other equivalent amino acids such as asparagine and of amino acid-like molecules.

The following examples give descriptions of stages in synthetic routes for preparing cationic polymers in accordance with the present invention for use in nucleic acid delivery vehicles in accordance with the present invention. They should not be construed as limiting the present invention.

Unless otherwise stated, molecular weight values quoted for polymers are intended to represent weight average values.

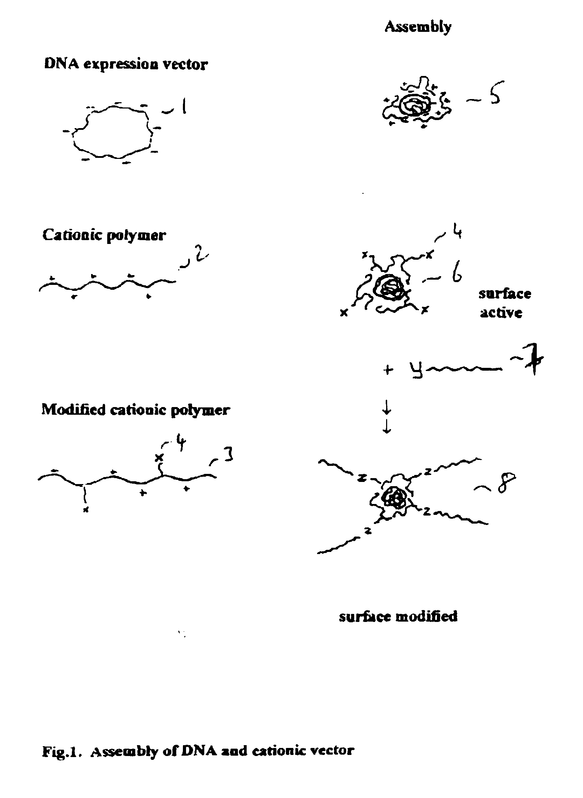

Fig. 1 is schematic representation of the main components and stages in the generation of a DNA vehicle in accordance with the present invention. A DNA expression vector 1, a cationic polymer 2 and a modified cationic polymer 3 provided with a plurality of reactive groups 4 along the polymer chain are brought together to form the DNA-containing core 6 with the exposed reactive groups 4 as a result of self-assembly. The assembly may be carried out as a two-stage process in which the DNA expression vector 1 is first self-assembled with the cationic polymer 2 to form a first complex 5. The cationic polymer 1 may include bioactive components such as fusogenic oleyl molecular groups for facilitating membrane penetration. The first complex 5 may then be brought into contact with the modified cationic polymer 3 to form the surface active second complex 5. A hydrophilic polymer 7 is then linked to the second complex 6 via the exposed reactive groups 4 to form a third complex 8, the hydrophilic polymer acting as a coating and an outer steric shield. By including biotin groups into the hydrophilic polymer 7, streptavidin/antibody conjugates may finally be attached to the complex 8 via the biotin groups to provide specific cell targeting groups.

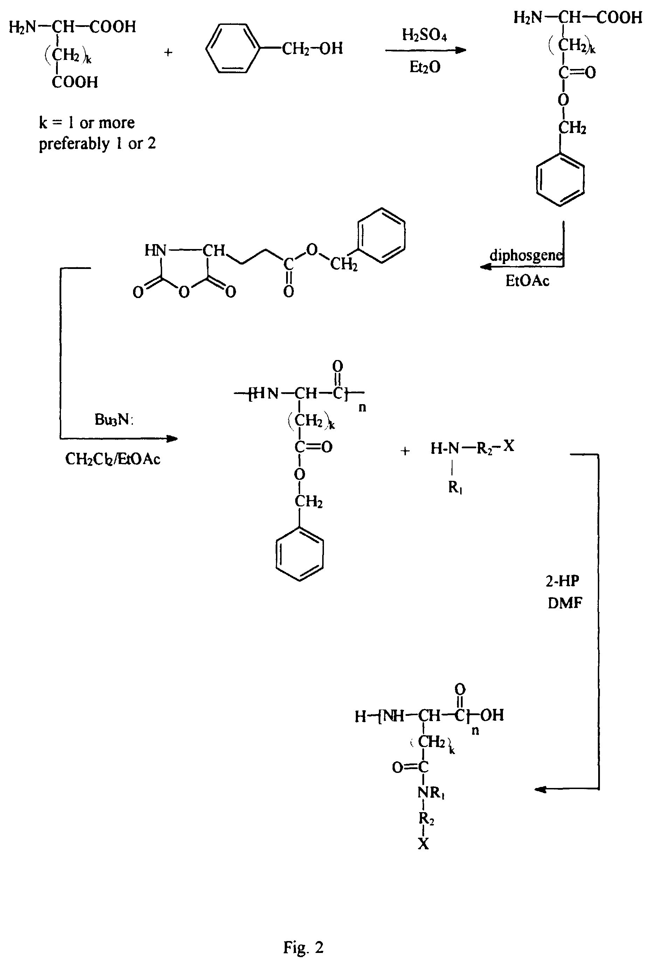

Fig. 2 is a schematic representation of a general synthesis scheme for a derivative of an amino acid or an amino acid-like molecule (k = 3 or above), in particular a derivative of glutamic or aspartic acid and in particular a poly[R₂N-R₁-X] glutamine or asparagine derivative in accordance with the present invention. The γ-carboxylic acid of L-glutamic acid, L-aspartic acid or equivalent amino acid or amino acid-like molecule is esterified in acidic medium with benzyl alcohol. From the γ-benzyl-L-glutamate or asparagate the corresponding N-carboxy anhydride (NCA) is prepared by reaction with phosgene or di-phosgene. The NCA is then polymerised using tributylamine as an initiator. Finally, poly-benzyl L-glutamate or -asparagate is aminolysed by reaction with the selected amine. For this aminolysis 2-hydroxy-pyridine can be used as a catalyst.

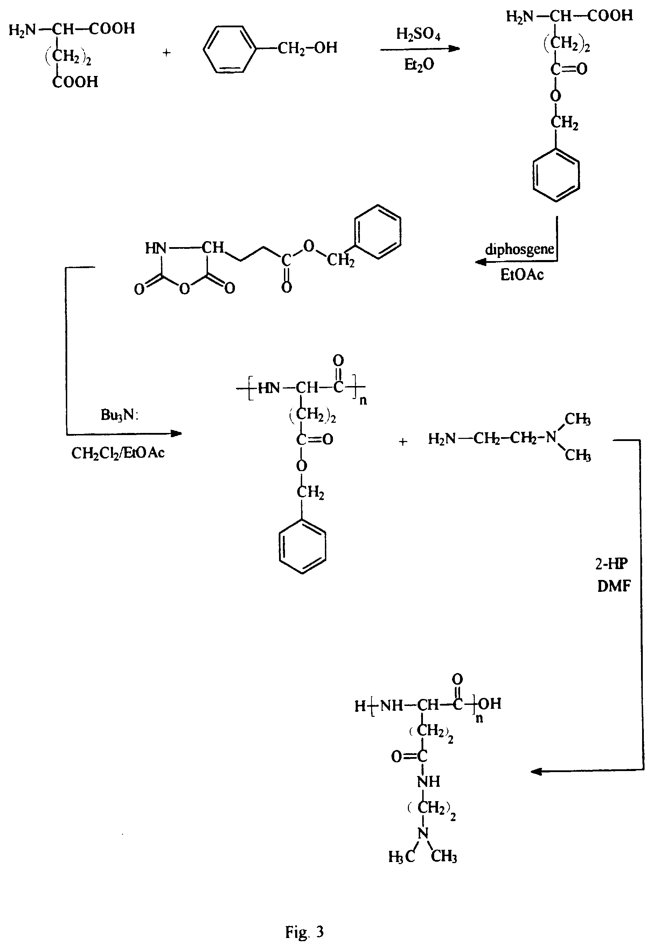

### Example 1: synthesis of poly(dimethylaminoethyl-L-glutamate) as shown schematically in Fig.3:

### Sub-step 1: synthesis of γ-benzyl-L-glutamate :

Sulphuric acid (98 %, 50 ml) is added dropwise, while vigorously stirring, to dry ether (500 ml) and cooled on an ice bath. Subsequently benzylic alcohol (500 ml) and L-glutamic acid (73,5 g, 0,5 mol) are added. The ether is evaporated at room temperature and the mixture is allowed to stand overnight, or longer, at room temperature. Afterwards 900 ml of water is added and the solution is neutralised on an ice bath with LiOH to pH = 6-6,5. The mixture is allowed to stand for another hour, followed by filtering off the precipitate and washing it ice cold water (2x) and ether (2x). The product is purified by recrystallization from an EtOH/H₂O mixture (9/1). The yield was 50 %.

### Sub-step 2: synthesis of the N-carboxyanhydride (NCA) derivative of γ-benzyl-L-glutamate :

γ-benzyl-L-glutamate (5 g, 0,0211 mol) is suspended in dry ethyl acetate (EtOAc) (50 ml). The suspension is put on an oil bath at 60 °C and a solution of diphosgene in EtOAc (5 % v/v) is added in portions of 5-10 ml. After approximately 15′ reaction, nitrogen (N₂) is flushed through the suspensions for about 2′ to remove the formed HCl and after this a new portion of diphosgene is added. This procedure is repeated until only a small amount of γ-benzyl glutamate is left. The oil bath is removed and the solution is flushed with N₂ for 30′. The suspension is filtered off and the filtrate is concentrate to about 75 ml. Dry hexane (15 ml) is added slowly until crystallization starts and is mixture is then put in the freezer for 1 hour. The white crystalline product is filtered off and washed with hexane. After drying the product is recrystallized from EtOAc until the product is sufficiently chloride free.
Chloride test: the NCA is boiled in 1 ml HNO₃ (2 M) until is completely dissolved. 1 ml AgNO₃ (0,05 M) is then added and when the turbidity is comparable to the one obtained in a similar experiment using tap water, the NCA is considered being sufficiently chloride free. The yield was 70 %.

### Sub-step 3: synthesis of poly-γ-benzyl-L-glutamate (PBG₃) :

The NCA of γ-benzyl-L-glutamate (3 g, 11,41 mmol) is dissolved in a mixture of CH₂Cl₂/EtOAc (18 ml/3 ml). Tributylamine (271 µl of a 10 % solution in CH₂Cl₂, for a M/I = 100/1) is added and the reaction is left overnight. During the reaction the mixture became viscous so another 35 ml of CH₂Cl₂ was added The polymer is received after precipitation in a MeOH/Et₂O (750 ml/375 ml) mixture, filtered off, washed with ether (3x) and dried under vacuum. The yield was 74 %.

### Sub-set 4: synthesis of poly(dimethylaminoethyl-L-glutamate) :

PBG (1g, 4,57 mmol esters) is dissolved in 10 ml dimethylformamide (DMF) and put on an oil bath at 40 °C. 2-dimethylaminoethylamine (10,02 ml, 91,3 mmol) and 2-hydroxypyridine (2,17 g, 22,8 mmol) are added and the solution is stirred for 48 hours. The reaction is followed by ¹H-NMR and infra red spectroscopy. The product is received after precipitation in excess ether (20x). Finally the product is dialysed against water for 2 days and freeze-dried. The yield was 60 %. The cationic polymer in accordance with this embodiment had a molecular weight of 59,240, was soluble in water and was protonated.

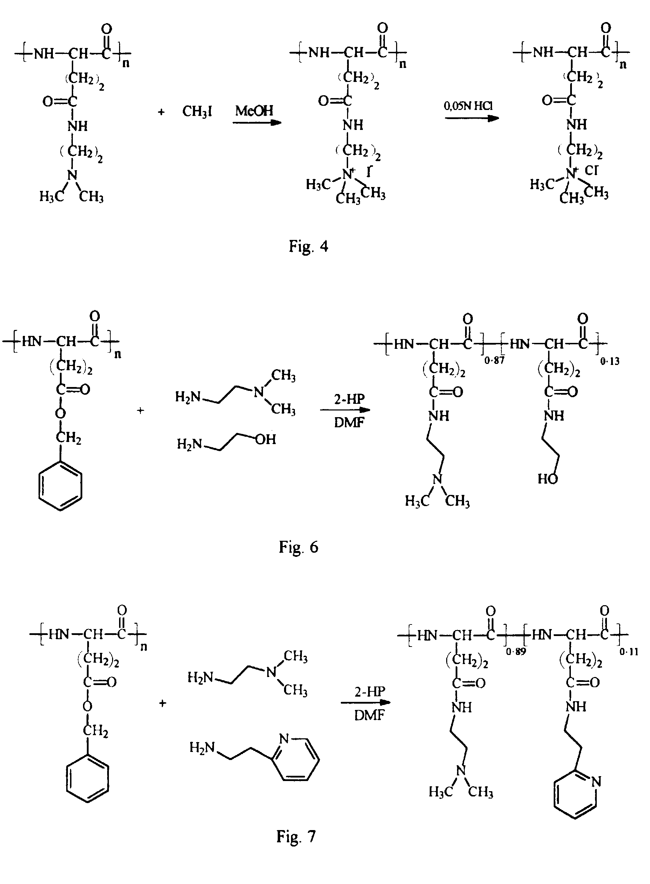

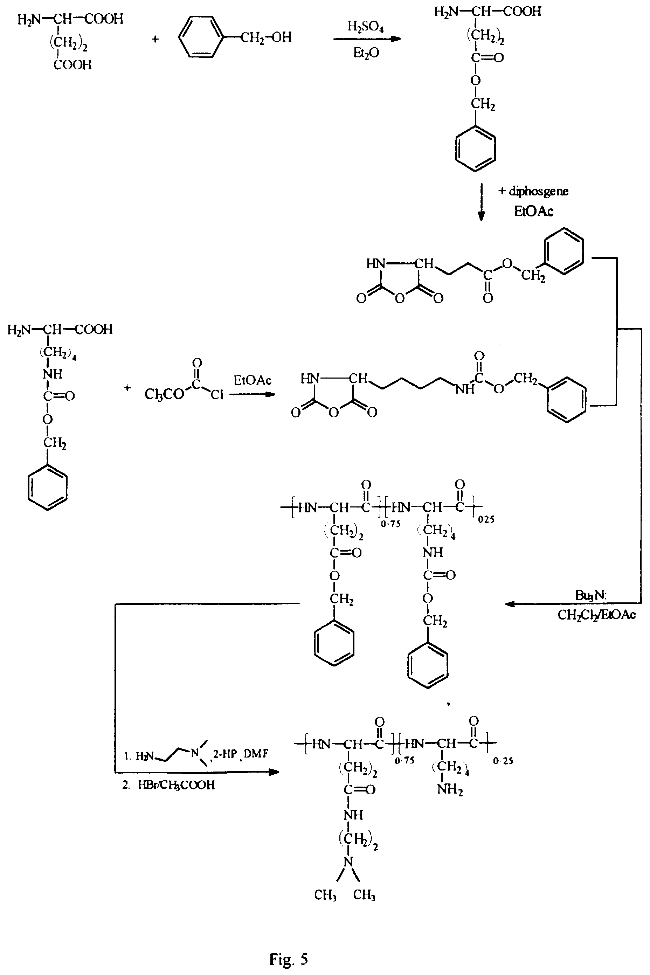

### Example 2: synthesis of poly(trimethylaminoethyl-L-glutamate) as shown schematically in Fig. 4:

Poly(dimethylaminoethyl-L-glutamate) (200 mg, 1 mmol tertiary amines) is dissolved in 12 ml MeOH. MeI (1,26 ml, 20 mmol) is added to this solution and the mixture is refluxed for one day. The MeI and MeOH are removed under reduced pressure. The product is resuspended in a 0,05 N HCl solution and dialysed against water for 2 days. The product is received after freeze-drying. The yield was 95 %. The cationic polymer in accordance with this embodiment had a molecular weight of 62,000, and was soluble in water.

### Example 3: synthesis of poly(dimethylaminoethyl-L-glutamate)-co-poly-L-lysine as shown schematically in Fig. 5:

### Sub-step 1: Synthesis of γ-benzyl-L-glutamate:

Sulphuric acid (98 %, 50 ml) is added dropwise, while vigorously stirring, to dry ether (500 ml) and cooled on an ice bath. Subsequently benzylic alcohol (500 ml) and L-glutamic acid (73,5 g, 0,5 mol) are added. The ether is evaporated at room temperature and the mixture is allowed to stand overnight, or longer, at room temperature. Afterwards 900 ml of water is added and the solution is neutralised on an ice bath with LiOH to pH = 6-6,5. The mixture is allowed to stand for another hour, followed by filtering off the precipitate and washing it ice cold water (2x) and ether (2x). The product is purified by recrystallization from an EtOH/H₂O mixture (9/1). The yield was 50%.

### Sub-step 2: synthesis of the N-carboxyanhydride (NCA) derivative of γ-benzyl-L-glutamate :

γ-benzyl-L-glutamate (5 g, 0,0211 mol) is suspended in dry ethyl acetate (EtOAc) (50 ml). The suspension is put on an oil bath at 60 °C and a solution of diphosgene in EtOAc (5 % v/v) is added in portions of 5-10 ml. After approximately 15′ reaction, nitrogen (N₂) is flushed through the suspensions for about 2′ to remove the formed HCl and after this a new portion of diphosgene is added. This procedure is repeated until only a small amount of γ-benzyl glutamate is left. The oil bath is removed and the solution is flushed with N₂ for 30′. The suspension is filtered off and the filtrate is concentrate to about 75 ml. Dry hexane (15 ml) is added slowly until crystallization starts and is mixture is then put in the freezer for 1 hour. The white crystalline product is filtered off and washed with hexane. After drying the product is recrystallized from EtOAc until the product is sufficiently chloride free.
Chloride test: The NCA is boiled in 1 ml HNO₃ (2 M) until is completely dissolved. 1 ml AgNO₃ (0,05 M) is then added and when the turbidity is comparable to the one obtained in a similar experiment using tap water, the NCA is considered being sufficiently chloride free. The yield was 70 %.

### Sub-step 3: synthesis of the N-carboxyanhydride derivative of ε-benzyloxycarbonyl-L-lysine :

ε-benzyloxycarbonyl-L-lysine (5 g, 0,0178 mol) is suspended in dry ethyl acetate (EtOAc) (50 ml). The suspension is put on an oil bath at 60 °C and a solution of diphosgene in EtOAc (5 % v/v) is added in portions of 5-10 ml. After approximately 15′ reaction, nitrogen (N₂) is flushed through the suspensions for about 2′ to remove the formed HCl and after this a new portion of diphosgene is added. This procedure is repeated until only a small amount of γ-benzyl glutamate is left. The oil bath is removed and the solution is flushed with N₂ for 30′. The suspension is filtered off and the filtrate is concentrate to about 75 ml. Dry hexane (15 ml) is added slowly until crystallization starts and is mixture is then put in the freezer for 1 hour. The white crystalline product is filtered off and washed with hexane. After drying the product is recrystallized from EtOAc until the product is sufficiently chloride free.
Chloride test: The NCA is boiled in 1 ml HNO₃ (2 M) until is completely dissolved. 1 ml AgNO₃ (0,05 M) is then added and when the turbidity is comparable to the one obtained in a similar experiment using tap water, the NCA is considered being sufficiently chloride free. The yield was 80 %.

### Sub-step 4: synthesis of poly(γ-benzyl-L-glutamate)_{0,75}-co-poly(ε-benzyloxycarbonyl-L-lysine_{0,25} (PBG₃-PLL₁) :

The NCA of γ-benzyl-L-glutamate (2,83 g, 10,77 mmol) and ε-benzyloxycarbonyl-L-lysine (1,1 g, 3,59 mmol) are dissolved in a mixture of CH₂Cl₂/EtOAc (24 ml/4 ml). Tributylamine (342 µl, for a M/I = 10/1) is added and the reaction is left overnight. The polymer is received after precipitation in a MeOH/Et₂O (280 ml/140 ml) mixture, filtered off, washed with ether (3x) and dried under vacuum. The yield was 80 %.

### Sub step 5: synthesis of poly(dimethylaminoethyl-L-glutamate)_{0,75}-co-poly(ε-benzyloxycarbonyl-L-lysine)_{0,25} :

PBG₃-PLL₁ (1g, 3,26 mmol esters) is dissolved in 10 ml dimethylformamide (DMF) and put on an oil bath at 40 °C. 2-dimethylaminoethylamine (7,17 ml, 65,3 mmol) and 2-hydroxypyridine (1,55 g, 16,3 mmol) are added and the solution is stirred for 70 hours. The reaction is followed by ¹H-NMR and infra red spectroscopy. The product is received after precipitation in ether (20x). Finally the product is dialyzed against water for 2 days and freeze-dried. The yield was 80 %.

### Sub-step 6: synthesis of poly(dimethylaminoethyl-L-glutamate)_{0,75}-co-poly(L-lysine)_{0,25} :

Poly(dimethylaminoethyl-L-glutamate)_{0,75}-co-poly(ε-benzyloxycarbonyl-L-lysine)_{0,25} (0,3 g) is dissolved in a minimal amount of acetic acid (CH₃COOH) and vigorously stirred. A same amount of a HBr/CH₃COOH solution is added and the mixture is stirred for 20′. The reaction is terminated through the addition of ice cold ether (12x). The precipitate is filtered off and washed with ether (3x). The crystals are resuspended in a 4 % NaHCO₃ solution, dialyzed against water for 2 days and finally the solution is freeze-dried. The yield was 60 %. The cationic polymer in accordance with this embodiment had a molecular weight of 77,000, was soluble in water and was protonated.

### Example 4: synthesis of poly(dimethylaminoethyl-L-glutamate_{87%}-co-poly(hydroxyethyl-L-glutamate)_{13%} as shown schematically in Fig. 6:

PBG (1 g, 4,57 mmol esters) is dissolved in 30 ml dimethylformamide (DMF) and put on an oil bath at 40 °C. 2-dimethylaminoethylamine (10,02 ml, 91,3 mmol) and 2-hydroxypyridine (2,17 g, 22,8 mmol) are added and the solution is stirred for 66 hours. The conversion, 75 %, was determined by ¹H-NMR. To complete the reaction 5 ml of aminoethanol is added, and the reaction is stirred for another 30 hours. The product is received after precipitation in ether (20x). Finally the product is dialysed against water for 2 days and freeze-dried. The yield was 60 %. The cationic polymer in accordance with this embodiment had a molecular weight of 92,000, was soluble in water and was protonated.

### Example 5: Synthesis of poly(dimethylaminoethyl-L-glutamate)_{89%}-co-poly(pyridinoethyl-L-glutamate)_{11%} as shown schematically in Fig. 7:

PBG (0,5 g, 2,28 mmol esters) is dissolved in 10 ml DMF and put on an oil bath at 45 °C. 2-dimethylaminoethylamine (5 ml, 46 mmol), 2-aminoethylpyridine (1,09 ml, 9,1 mmol) and 2-hydroxypyridine (1,08 g, 11,4 mmol) are added and the solution is stirred for 60 hours. The product is received after precipitation in ether (20x). Finally the product is dialysed against water for 2 days and freeze-dried. The molecular weight of the cationic polymer in accordance with this embodiment was 60,000, was protonated and was soluble in water. The ratio of dimethylaminoethyl-L-glutamate molecular units to the pyridino-ethyl-L-glutamate molecular units in the copolymer was 89:11.

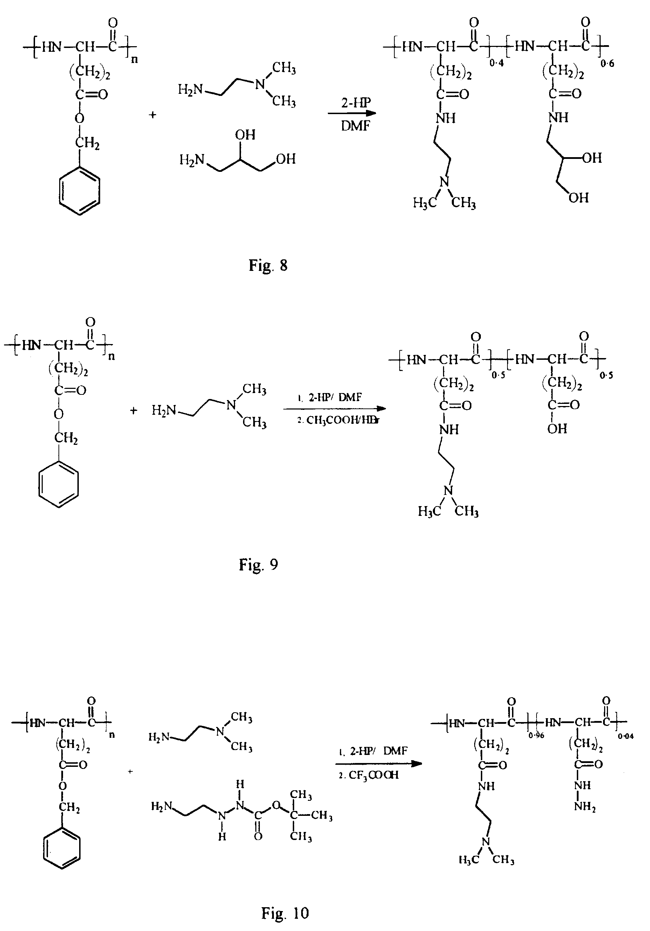

### Example 6: synthesis of poly(dimethylaminoethyl-L-glutamate_{40%}-co-poly(dihydroxypropyl-L-glutamate)_{60%} as shown schematically in Fig. 8:

PBG (0,5 g, 2,28 mmol esters) is dissolved in 10 ml DMF and put on an oil bath at 50 °C. 2-dimethylaminoethylamine (2,5 ml, 23 mmol), 3-amino-1,2-propanediol (1,76 ml, 23 mmol) and 2-hydroxypyridine (1,08 g, 11,4 mmol) are added and the solution is stirred for 70 hours. The product is received after precipitation in ether (20x). Finally the product is dialysed against water for 2 days and freeze-dried. The molecular weight of the cationic polymer in accordance with this embodiment was 98,000. It was soluble in water and protonated.

### Example 7: synthesis of poly(dimethylaminoethyl-L-glutamate)_{50%}-co-poly-L-glutamic acid_{50%} as shown schematically in Fig. 9:

### Sub-step 1: aminolysis of the benzylic esters :

PBG (0,5 g, 2,28 mmol esters) is dissolved in 10 ml DMF and put on an oil bath at 40 °C. 2-dimethylaminoethylamine (2,5 ml, 23 mmol) and 2-hydroxypyridine (1,08 g, 11,4 mmol) are added and the solution is stirred for 20 hours. The product is received after precipitation in ether (20x). Finally the product is dialysed against water for 2 days and freeze-dried. By H-NMR we can calculate that we have 50 % conversion of the esters.

### Sub-step 2: removal of the remaining benzylic esters :

To a solution of poly(dimethylaminoethyl-L-glutamate)_{50%}-co-poly(PBG)_{50%} (0,25 g) in acetic acid (25 ml), is added a same amount of a 33 % HBr/CH₃COOH solution. The mixture is stirred for 15′-20′ and is than poured in ice cold ether (500 ml). The crystals are filtered off and washed with ether, resuspended in a 4 % NaHCO₃ solution, dialysed against water, and finally freeze-dried.

### Example 8: Synthesis of poly(dimethylaminoethyl-L-glutamate)_{96%}-co-poly(hydrazido-L-glutamate)_{4%} as shown schematically in Fig. 10:

### Sub-step 1: synthesis of poly(dimethylaminoethyl-L-glutamate)_{96%}-co-poly(t.boc-hydrazido-L-glutamate) :

PBG (0,5 g, 2,28 mmol esters) is dissolved in 10 ml DMF and put on an oil bath at 40 °C. 2-dimethylaminoethylamine (5 ml, 46 mmol), t.-butylcarbazate (1,2 g, 9,12 mmol) and 2-hydroxypyridine (1,08 g, 11,4 mmol) are added and the solution is stirred for 48 hours. The product is received after precipitation in ether (20x). Finally the product is dialysed against water for 2 days and freeze-dried.

### Sub-step 2: removal of the hydrazide protecting group :

Poly(dimethylaminoethyl-L-glutamate)_{96%}-co-poly(t.boc-hydrazido-L-glutamate) (340 mg) is dissolved in a mixture of trifluoro acetic acid (6 ml) and water (2 ml), and stirred for 2 hours at room temperature. The solvent is removed under reduced pressure and the product is redissolved in water and dialysed against a 0,05 N HCl solution and water. The final product is received after freeze-drying.

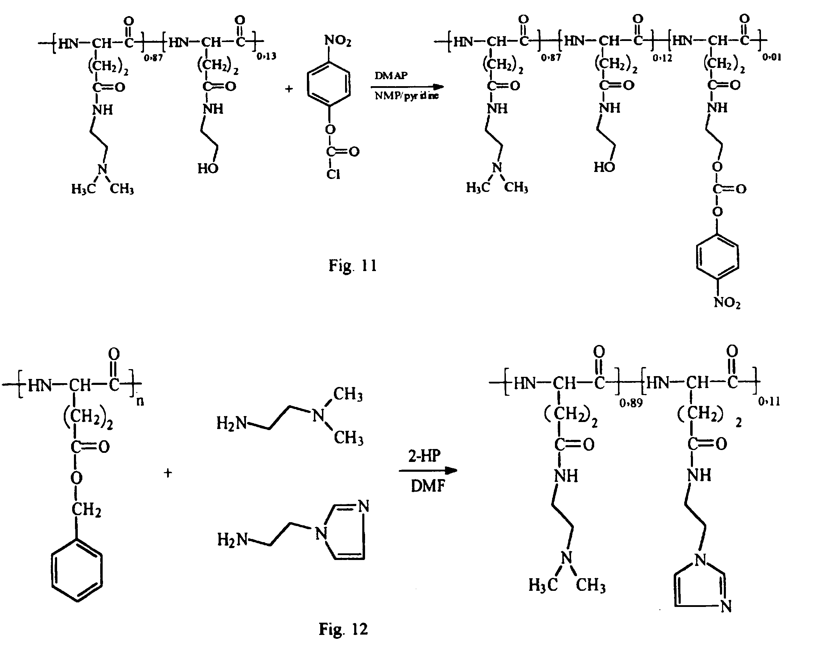

### Example 9: p-nitrophenyl activation of poly(dimethylaminoethyl-L-glutamate)_{85%}-co-poly(hydroxyethyl -L-glutamate)_{15%} as shown schematically in Fig. 11:

Poly(dimethylaminoethyl-L-glutamate)_{87%}-co-poly(hydroxyethyl-L-glutamate)_{13%} (0.1 g, 0,0663 mmol alcohol functions) and 4-dimethylaminopyridine (0,73 mg, 0,006 mmol) were dissolved in 4 ml NMP and 1 ml pyridine, and chloroformate (8,61 mg, 0,04 mmol) was added at 0 °C. After 4 hours reaction at 0 °C the reaction was precipitated in a mixture of anhydrous ether. The precipitate was collected and washed repeatedly with ether until all choroformate was removed. The product (78,3 mg) was dissolved in dimethylsulphoxide (2,5 ml) and stored in the freezer. The carbonate content, determined by UV analysis after alkaline hydrolysis in NaOH (λ_{M} = 402 nm, ε_{M} = 18400 l mmol⁻¹ cm^{-¹}), was 8,09% of the alcohol functions (= 1 % per chain).

### Example 10: synthesis of poly(dimethylaminoethyl-L-glutamate)_{89%}-co-poly(imidazoloethyl-L-glutamate)_{11%} as shown schematically in Fig. 12:

PBG (0,5 g, 2,28 mmol esters) is dissolved in 10 ml DMF and put on an oil bath at 45 °C. 2-dimethylaminoethylamine (5 ml, 46 mmol), 2-aminoethylimidazol (1,01 g, 9,1 mmol) and 2-hydroxypyridine (1,08 g, 11,4 mmol) are added and the solution is stirred for 65 hours. The product is received after precipitation in ether (20x). Finally the product is dialysed against water for 2 days and freeze-dried.

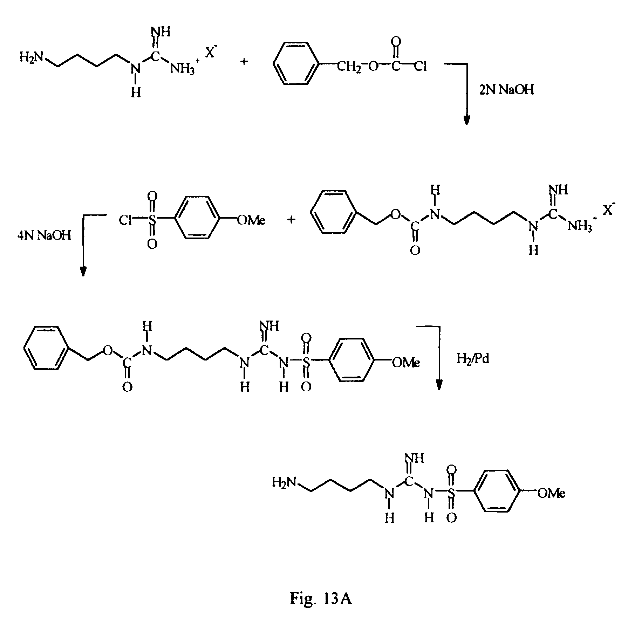

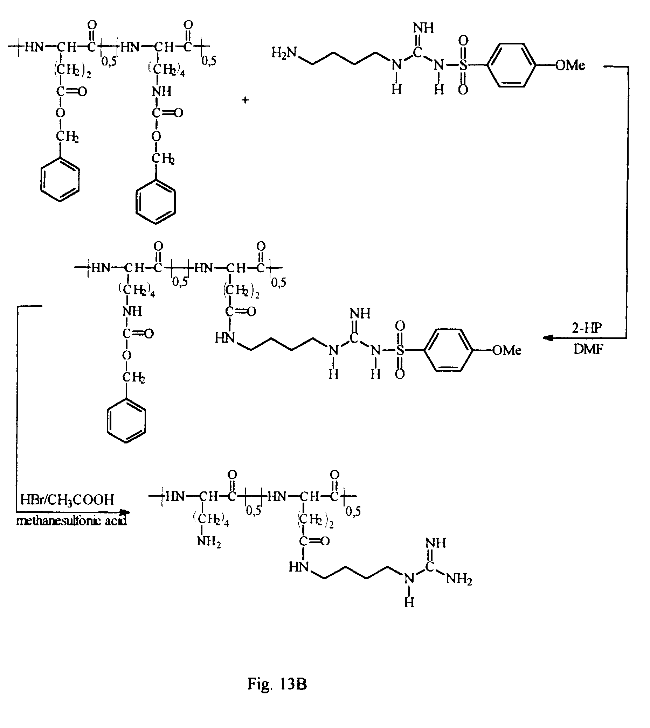

### Example 11: Synthesis of polyagmatino-L-glutamate_{0,5}-co-poly-L-lysine_{0,5} as shown schematically in Figs. 13A and B:

### Sub-step 1: synthesis of benzyloxycarbonyl protected agmatine :

Agmatine sulphate (3 g; 13,14 mmol) is suspended in ice cold 2 N NaOH (10 ml). The solution is cooled to 0 °C and stirred. Benzyloxycarbonyl chloride (2,438 ml; 17, 08 mmol) and 2 N NaOH are alternately added in portions (pH is kept between 9 and 9,5). The mixture is stirred for another 2 hours (pH drops to 7). The precipitate is filtered off and the filtrate is extracted with ether (3x). The aqueous phases are freeze-dried.

### Sub-step 2: synthesis of N^{α}-benzyloxycarbonyl-N^{G}-p-methoxybenzenesulfonylagmatine:

Benzyloxycarbonyl protected agmatine (700 mg; 2,65 mmol) is suspended in 4 N NaOH (2,5 ml) and acetone (22 ml) at room temperature. Water is added until the solution becomes clear. The solution is cooled to 0 °C and a solution of p-methoxybenzenesulphonyl chloride (780 mg; 3,79 mmol in 5 ml acetone) is added dropwise during 30 minutes. The solution is stirred for 2 hours at 0°C and for 2 hours at room temperature. The filtrate is removed by means of a pipette (pH = 11). Citric acid is added to make the solution acidic. The solvent is removed under reduced pressure. The residue is resuspended in 20 ml water (pH = 2-3) and extracted with EtOAc (4x). The organic phase is washed with distilled water, followed by purification of silica (eluens is EtOAc). The product is received after drying under vacuum for 1 day.

### Sub-step 3: synthesis of p-methoxybenzenesulphonyl protected agmatine :

N^{α}-benzyloxycarbonyl-N^{G}-p-methoxybenzenesulphonyl-agmatine (25 mg; 0,6 mmol) is dissolved in 1 ml MeOH. Pd/C (10 mg) is added and the mixture is put under a H₂ atmosphere (H₂ balloon). The mixture is stirred overnight. The catalyst is filtered over celiet and the filtrate is removed under reduced pressure. The product is dried under vacuum

### Sub-step 4: synthesis of polyagmatino-L-glutamate_{0,5}-co-poly-ε-benzyloxycarbonyl-L-lysine_{0,5} :

Poly-γ-benzyl-L-glutamate_{0,5}-co-poly-ε-benzyloxycarbonyl-L-lysine_{0,5} (0,5 g; 1,1 mmol) is dissolved in a 10 ml DMF and put on an oil bath at 45 °C. p-methoxybenzenesulphonyl protected agmatine (9,5 g; 22 mmol) and 2-hydroxypyridine (523 mg; 5,5 mmol) are added and the solution is stirred for 65 hours. The product is received after precipitation in ether (20x). Finally the product is dialysed against water for 2 days and freeze-dried.

### Sub-step 5: synthesis of polyagmatino-L-glutamate_{0,5}-co-poly-L-lysine_{0,5}:

Polyagmatino-L-glutamate_{0,5}-co-poly-ε-benzyloxycarbonyl-L-lysine_{0,5} (0,3 g) is dissolved in a minimal amount of acetic acid and vigorously stirred. A mixture of a HBr/CH₃COOH solution and methane sulphonic acid containing anisole, is added and the reaction mixture is stirred at room temperature for 30 minutes. The reaction is terminated through the addition of ice cold ether (20x). Finally the product is dialysed against water for 2 days and freeze-dried. The yield was 80 %.

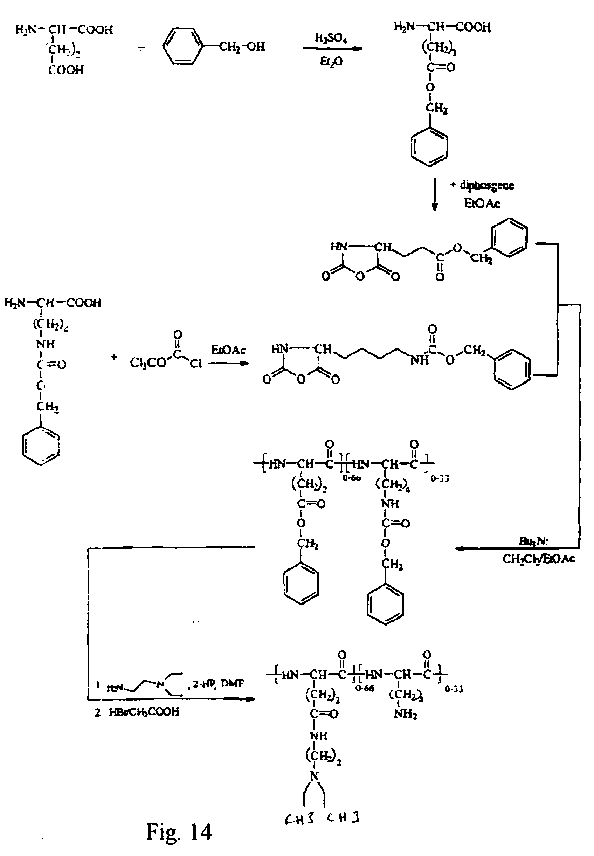

### Example 12: Synthesis of poly(diethylaminoethyl-L-glutamate)_{0,66}-co-poly-L-lysine_{0,33} as shown schematically in Fig. 14:

### Sub-step 1: Synthesis of γ-benzyl-L-glutamate:

Sulphuric acid (98 %, 50 ml) is added dropwise, while vigorously stirring, to dry ether (500 ml) and cooled on an ice bath. Subsequently benzylic alcohol (500 ml) and L-glutamic acid (73,5 g, 0,5 mol) are added. The ether is evaporated at room temperature and the mixture is allowed to stand overnight, or longer, at room temperature. Afterwards 900 ml of water is added and the solution is neutralised on an ice bath with LiOH to pH = 6-6,5. The mixture is allowed to stand for another hour, followed by filtering off the precipitate and washing it ice cold water (2x) and ether (2x). The product is purified by recrystallization from an EtOH/H₂O mixture (9/1). The yield was 50 %.

### Sub-step 2: synthesis of the N-carboxyanhydride (NCA) derivative of γ-benzyl-L-glutamate :

γ-benzyl-L-glutamate (5 g, 0,0211 mol) is suspended in dry ethyl acetate (EtOAc) (50 ml). The suspension is put on an oil bath at 60 °C and a solution of diphosgene in EtOAc (5 % v/v) is added in portions of 5-10 ml. After approximately 15′ reaction, nitrogen (N₂) is flushed through the suspensions for about 2′ to remove the formed HCl and after this a new portion of diphosgene is added. This procedure is repeated until only a small amount of γ-benzyl glutamate is left. The oil bath is removed and the solution is flushed with N₂ for 30′. The suspension is filtered off and the filtrate is concentrate to about 75 ml. Dry hexane (15 ml) is added slowly until crystallization starts and is mixture is then put in the freezer for 1 hour. The white crystalline product is filtered off and washed with hexane. After drying the product is recrystallized from EtOAc until the product is sufficiently chloride free.
Chloride test: The NCA is boiled in 1 ml HNO₃ (2 M) until is completely dissolved. 1 ml AgNO₃ (0,05 M) is then added and when the turbidity is comparable to the one obtained in a similar experiment using tap water, the NCA is considered being sufficiently chloride free. The yield was 70 %.

### Sub-step 3: synthesis of the N-carboxyanhydride derivative of ε-benzyloxycarbonyl-L-lysine :

ε-benzyloxycarbonyl-L-lysine (5 g, 0,0178 mol) is suspended in dry ethyl acetate (EtOAc) (50 ml). The suspension is put on an oil bath at 60 °C and a solution of diphosgene in EtOAc (5 % v/v) is added in portions of 5-10 ml. After approximately 15′ reaction, nitrogen (N₂) is flushed through the suspensions for about 2′ to remove the formed HCl and after this a new portion of diphosgene is added. This procedure is repeated until only a small amount of γ-benzyl glutamate is left. The oil bath is removed and the solution is flushed with N₂ for 30′. The suspension is filtered off and the filtrate is concentrate to about 75 ml. Dry hexane (15 ml) is added slowly until crystallization starts and is mixture is then put in the freezer for 1 hour. The white crystalline product is filtered off and washed with hexane. After drying the product is recrystallized from EtOAc until the product is sufficiently chloride free.
Chloride test: The NCA is boiled in 1 ml HNO₃ (2 M) until is completely dissolved. 1 ml AgNO₃ (0,05 M) is then added and when the turbidity is comparable to the one obtained in a similar experiment using tap water, the NCA is considered being sufficiently chloride free. The yield was 80 %.

### Sub-step 4: synthesis of poly(γ-benzyl-L-glutamate)_{0,66}-co-poly(ε-benzyloxycarbonyl-L-lysine)_{0,33}(PBG₂-PLL₁) :

The NCA of γ-benzyl-L-glutamate (2,83 g, 10,77 mmol) and ε-benzyloxycarbonyl-L-lysine (1,6 g, 5,04 mmol) are dissolved in a mixture of CH₂Cl₂/EtOAc (24 ml/4 ml). Tributylamine (342 µl, for a M/I = 10/1) is added and the reaction is left overnight. The polymer is received after precipitation in a MeOH/Et₂O (280 ml/140 ml) mixture, filtered off, washed with ether (3x) and dried under vacuum. The yield was 80 %.

### Sub step 5: synthesis of poly(diethylaminoethyl-L-glutamate)_{0,66}-co-poly(ε-benzyloxycarbonyl-L-lysine)_{0,33} :

PBG₂-PLL₁ (1g, 2,9 mmol esters) is dissolved in 10 ml dimethylformamide (DMF) and put on an oil bath at 55 °C. 2-diethylaminoethylamine (8,2 ml, 58 mmol) and 2-hydroxypyridine (1,38 g, 14,5 mmol) are added and the solution is stirred for 75 hours. The reaction is followed by ¹H-NMR and infra red spectroscopy. The product is received after precipitation in ether (20x). Finally the product is dialyzed against water for 2 days and freeze-dried. The yield was 80 %.

### Sub-step 6: synthesis of poly(diethylaminoethyl-L-glutamate)_{0,66}-co-poly(L-lysine)_{0,33} :

Poly(diethylaminoethyl-L-glutamate)_{0,66}-co-poly(ε-benzyloxycarbonyl-L-lysine)_{0,33} (0,3 g) is dissolved in a minimal amount of acetic acid (CH₃COOH) and vigorously stirred. A same amount of a HBr/CH₃COOH solution is added and the mixture is stirred for 20′. The reaction is terminated through the addition of ice cold ether (12x). The precipitate is filtered off and washed with ether (3x). The crystals are resuspended in a 4 % NaHCO₃ solution, dialyzed against water for 2 days and finally the solution is freeze-dried. The yield was 60 %. The cationic polymer in accordance with this embodiment had a molecular weight of 77,000, was soluble in water and was protonated.

### Ethidium bromide condensation tests :

By performing this test, information can be obtained about the ability of the polymers to condense DNA. Formation of complexes can be followed by looking at the loss of fluorescence of EtBr at a wavelength of 590 nm. The samples contained 20 µg/ml of DNA and 400 ng/ml of EtBr. The polymers were added in a stepwise addition up to a about a 2:1 charge ratio and the solution was mixed by means of a Vortex. The fluorescence was recorded at wavelengths of λₑₓ = 366 nm and λₑₘ = 590 nm. A sample containing 400 ng/ml EtBr was used as a blank, this is 0 % fluorescence, the one containing 20 µg/ml DNA and 400 ng/ml EtBr was used to calibrate the machine to 100% fluorescence. The tests were performed at room temperature using a Perkin Elmer luminescence spectrometer LS 50B.

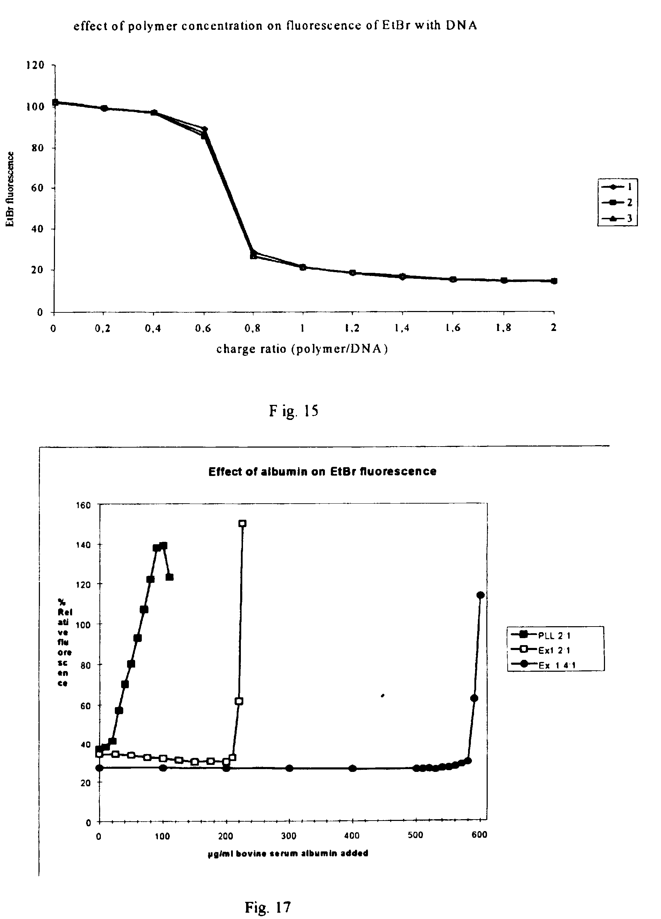

With all the examples, the MW of the amine = 249 g/mol versus 325 g/mol for the DNA. Fluorescence was measured 3 minutes after addition of polymer. The results are shown in Fig. 15. All the polymers were able to condense DNA. Condensation seems to appear optimal at a charge ratio of about 0.8:1, except for example 12 where the condensation seems to appear at a charge ratio of about 0.6:1.

### Transfection studies of 293 cells :

293 cells are human kidney cells and were selected as they are considered to be easily transfectable.

### Experimental procedure:

Dividing of the 293 cells:
   Fresh medium was prepared from a mixture of 0,5 L DME, 50 ml FCS and 5 ml glutamine. Trypsine solution was prepared from 1 bottle of EDTA, 1 bottle of trypsine and 1 bottle of 2x DUBELCA (mainly a PBS solution). The bottle containing the 293 cells was taken out of the incubator. The medium was removed. The cells were washed with 10 ml DUBELCA (mainly a PBS solution). The PBS was removed. 4 ml of the trypsine solution were added and left it on the cells for 1 minute. The trypsine solution was then removed. The bottle was put back in the incubator for 5 minutes to allow the cells to detach from the wall of the bottle. The bottle may be hit gently to detach the cells. 10 ml of the freshly prepared medium are then added. The medium is then separated into two new bottles and medium added so that the total amount of medium in each bottle is 10 ml. The bottles are returned to the incubator.
Plating out 293 cells for transfection studies :
   Remark : this should be performed 24 hours before doing the tests. Fresh medium was prepared from a mixture of 0,5 L DME, 50 ml FCS and 5 ml glutamine. The trypsine solution was prepared from 1 bottle of EDTA, 1 bottle of trypsine and 1 bottle of 2x DUBELCA. The bottle containing the 293 cells was taken out of the incubator and the medium removed. The cells were washed with 10 ml DUBELCA and the PBS removed 4 ml of the trypsine solution was added and left it on the cells for 1 minute. Then the trypsine solution was removed. The bottle was put back in the incubator for 5 minutes to allow the cells to detach from the wall of the bottle. The bottle may be hit gently to detach the cells. Then 10 ml of the freshly prepared medium was added. A small amount of this solution removed, which was used to count the cells. 50 µl cells + 50 µl tryphan blue + 100 µl PBS were mixed and a small amount of this solution was placed on a haemocytometer and the number of living cells coumted. The cells that are dead have take up the dye. The cells were then put in a 96 well plate so that every well contains about 8500 cells.
Preparation of samples :
   A DNA solution was prepared using is a pCMV P plasmid and a stock solution prepared having 2100 µg/ml. This was diluted so that the final concentration was 200 µg/ml. Each well contained in the end about 20 µg/ml. For each candidate polymer in accordance with the present invention a solution was prepared and a stock solution prepared of 200 µl/ml. Each well contained the polymer in a 2:1 charge ratio. DOTAP solution, a commercial liposomal transfection reagent, was used as a reference. A stock solution was prepared including 1000 µg/ml. The wells contained in the end 120 µg/ml.
Transfection of 293 cells :
   A fresh medium A was prepared from 0,5 L DME, 5 ml glutamine, and chloroquine (100 µM). A fresh medium B was prepared from 0,5 L DME, 50 ml FCS and 5 ml glutamine. The 96 well plate was removed from incubator. The cells were washed with PBS. 200 µl of medium A + 30 µl of the preformed samples were put in each well. The 96 well plates were returned to the incubator for 5 hours. Afterwards the medium was removed from the wells. The cells were washed with PBS. 200 µl of medium B were added to each cell and incubated for 48 hours.

### Evaluation of gene expression :

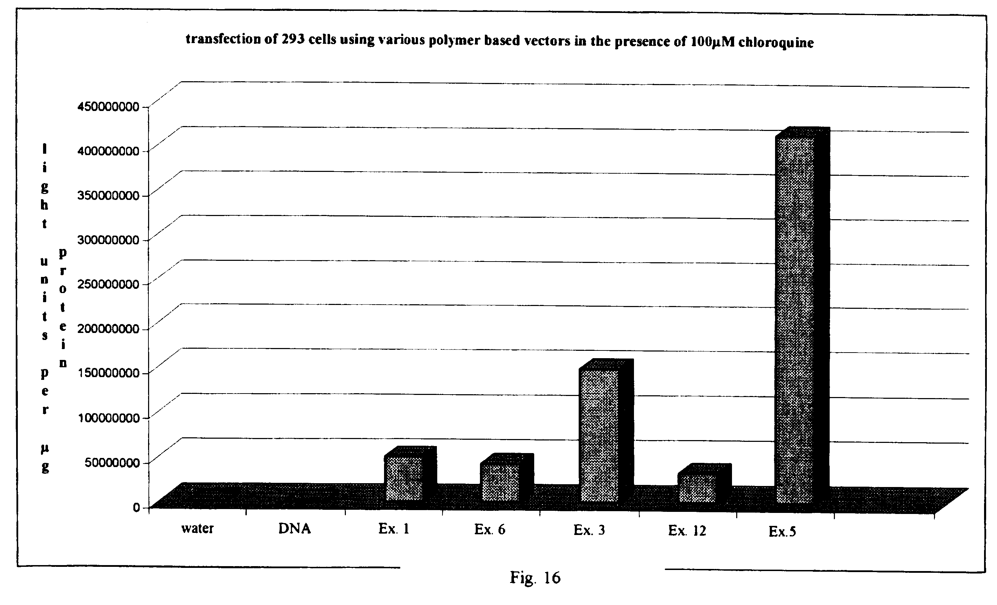

Expression of β-galactosidase was assessed histologically by washing the cells with PBS and fixing with formaldehyde (2%), glutaraldehyde (0,2 %) in PBS (4 °C, 5 min). The cells were washed again with PBS and incubated with X-gal substrate solution (1 mg/ml X-gal reagent, 5 mM K₃Fe(CN)₆, 5 mM K₄Fe(CN)₆, mM MgCl₂ in PBS) at 37 °C for at least 12 hours. X-gal (5-bromo-4-chloro-3-indolyl-β-D-galactopyranoside) is a chromogenic substrate for β-galactosidase, which yields a blue precipitate. After a final wash stage with PBS the cells were examined by light microscopy. Total β-galactosidase activity was quantified using the chemiluminiscent assay system Galacto-Light-Plus™. Cells were lysed in 200 µl lysis solution (100 mM potassium phosphate pH 7,8; 0,2 % Triton X-100, 1 mM dithiotreitol) by three freeze-thaw cycles of the entire 96-well plate. After centrifugation, 50 µl of the supernatant was then added to 200 µl chemiluminiscent substrate solution (Galacton-Plus™ in 100 mM sodium phosphate pH 8, 1 mM magnesium chloride). After incubation for 1 hour, 300 µl luminiscence accelerator reagent was added and the sample was assayed on a monolight luminometer (Lumat LB9501, Berthold) by counting emitted light units for 10 seconds. Transfection was quantified as β-galactosidase activity measured per µg protein (light units/µg). The total protein content was measured using the bicinconninic acid (BCA) assay for protein. BCA assay for protein was carried out in the following way: cell lysates and a series of protein standard solutions prepared with final bovine serum albumin in lysis buffer (0 to 500 µg/ml) were assayed (50 µl) for total protein. BCA reagent (200 µl) was added to each sample and after 30 minutes at 37 °C. The absorbance was read at a wavelength of 550 nm in an automatic microtitre plate spectrophotometer. The results are shown in Fig. 16. The highest levels are received when using example 5. A possible non-limiting reason for this result is that the pyridine group of example 5 causes an extra buffering of the endosome, allowing the DNA/cationic polymer complexes to be released from the endosome.

### Stability in presence of bovine serum albumin BSA (Sigma)

Complexes were made in charge ratios 2/1 and 4/1 and allowed to stabilize for 90 minutes. Baseline fluorescence was recorded and aliquot portions of BSA were gradually added. The results are shown in Fig. 17. For poly-L-lysine complexes (PLL) with a charge ratio of 2/1 the fluorescence increases significantly after addition of 30 µg/ml BSA. However, no such increase was observed for Ex. I according to the present invention even after addition of 200 µg/ml or 600 µg/ml, respectively for charge ratios of 2/1 and 4/1. It is important to note that the change in fluorescence in these cases was due to flocculation and precipitation of the complexes and did not result from ethidium bromide uptake in the DNA. It can be concluded that the DNA complexes in accordance with the present invention are much more stable than the corresponding PLL ones.

## Claims

**1.** A method of constructing a synthetic polymer-based carrier vehicle for delivery of nucleic acid material to target cells in biological systems, said method comprising the steps of:
a) providing a cationic polyelectrolyte polymer material including a
poly 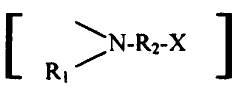 derivative of an amino acid or a synthetic amino acid; and
b) bringing the nucleic acid material into association with said cationic polyelectrolyte polymer material to form by self-assembly therebetween a polyelectrolyte complex which provides a nucleic acid containing cationic polymer core for said carrier vehicle.

**2.** For delivery of DNA to target cells in biological systems, a synthetic polymer-based carrier vehicle that comprises a polyelectrolyte complex in which a plasmid DNA expression vector located in a core portion is electrostatically bound and condensed through self-assembly with a polycationic polymer including a
poly 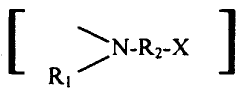 derivative of an amino acid or a synthetic amino acid.

**3.** A synthetic polymer-based carrier vehicle for delivery of nucleic acid to target cells in biological systems wherein said carrier vehicle is in the form of a particle consisting of a polyelectrolyte complex comprising a nucleic acid molecule bound to one or more molecules of cationic polymer material thereby forming a nucleic acid-containing core, said polyelectrolyte complex including a
poly 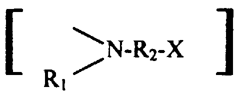 derivative of an amino acid or a synthetic amino acid.

**4.** A method according to claim 1 or a vehicle according to claim 2 or 3, wherein the amino acid is glutamic or aspartic acid.

**5.** A method according to claim 1 or 4, or a vehicle according to claim 3 or 4, wherein the cationic polyelectrolyte polymer material includes molecules of 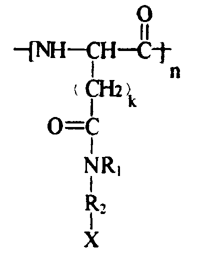 Where k is 1 or more, preferably 1 or 2.

**6.** A method or vehicle according to any previous claim where the X group is an amine.

**7.** A method or vehicle according to any previous claim, wherein the X group is a group with the general formula 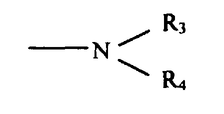 where R_{3,} R₄ are selected from hydrogen, an alkyl group or an alkylaryl group and R₃ does not have to be the same as R₄, the alkyl group may be selected from a methyl, ethyl, propyl, group or higher orders or the aryl group may be selected from a phenyl, napthyl, anthryl group, or higher orders.

**8.** A method or vehicle according to any previous claim wherein the X group is a pyridino- group having the general formula: 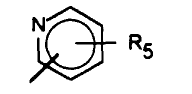 where R₅ may be selected from hydrogen or an alkyl group such as methyl, ethyl, propyl group or higher orders.

**9.** A method or vehicle according to any previous claim, wherein the X group may be an imidazolido- group of the general formula: 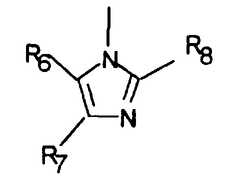 where R₆, R₇, R₈ may be selected from hydrogen or an alkyl group such as a methyl, ethyl, propyl group or higher orders and R₆, R₇, R₈ do not have to be the same.

**10.** A method or vehicle according to any previous claim, wherein R₁ may be selected from hydrogen, or an alkyl, aryl, or alkylaryl group, whereby the alkyl can be methyl, ethyl, propyl, or higher orders, and the aryl group may be a phenyl, naphyl, anthryl group, or higher orders and R₂ may be selected from any alkyl group with the general formula (CH₂)ₘ where m can be any value including 2 or greater, in particular any value between 2 and 20.

**11.** A method or vehicle according to any previous claim wherein the cationic polyelectrolyte polymer material includes molecules of a poly[N-alkyl-N-R,R'] glutamine or asparagine derivative.

**12.** A method according to any previous claim, further comprising the step of reacting said cationic polyelectrolyte polymer material or said polyelectrolyte complex with reactive hydrophilic polymer material so that the latter is included in said complex and forms a hydrophilic coating that provides an outer protective steric shield and assists in stabilising the complex.

**13.** A vehicle according to any of claims 2 to 11, wherein said core is coupled via covalent linkages to one or more associated hydrophilic polymer molecules forming a stabilising and protective steric shield or coating around said core, and wherein one or more other molecular entities providing bioactive agents or cell receptor targeting moieties are coupled, also via covalent linkages, to said cationic polymer material and/or to the hydrophilic polymer material, with at least some of said covalent linkages being hydrolytically unstable and/or pH sensitive or enzymatically sensitive so as to be degradable within the intracellular environment following endocytic uptake and internalisation by a target cell.

**14.** A method according to claim 12, wherein the molecules of both the hydrophilic polymer material and the cationic polyelectrolyte polymer material are formed or provided with reactive groups that react to attach or link the hydrophilic polymer material to the cationic polymer material by way of covalent bonds.

**15.** A method according to claim 12 or 14 or a vehicle according to claim 13, wherein specific cell targeting groups are provided attached to the cationic polymer core or to the hydrophilic coating polymer material.

**16.** A method or vehicle according to claim 15 wherein the targeting groups are selected from growth factors, antibodies and transferrin.

**17.** A method to any of claims 1, 4 to 12, 14 to 16 or a vehicle according to any of the claims 2 to 11, 13, 15, 16, wherein the cationic polyelectrolyte polymer molecules have a molecular weight in the range of 3 kDa to 1000 kDa, preferably 10 kDa to 200 kDa.

**17.** A method or a vehicle according to any of the previous claims, wherein the cationic polyelectrolyte polymer material is a polymer or copolymer composed of monomers selected from L- and D- glutamine and other amino acids.

**18.** A method or vehicle according to any of the previous claims, in which the charge ratio of the cationic polyelectrolyte polymer material to nucleic acid is within the range 0.5 to 5, preferably 0.5 to 2.

**19.** A method or vehicle as claimed in any of the preceding claims, wherein a fusogenic peptide or lipid based membrane disrupting agent is incorporated in the polyelectrolyte complex before reacting with the hydrophilic polymer material.

**20.** A method or vehicle as claimed in any of the previous claims, wherein the nucleic acid material is selected from any of DNA fragments, genomic DNA, plasmid DNA, cDNA, RNA, antisense nucleic acid and oligonucleotides.

**21.** A method or vehicle as claimed in any of the previous claims, wherein the cationic polyelectrolyte polymer material is pH responsive such that its degree of protonation changes in an acidic environment as occurs in the endosomal compartment of mammalian cells.

**22.** A pharmaceutical composition containing as the active material nucleic acid carrier vehicles as claimed in any of the claims 2 to 11, 13 to 21.

**23.** A synthetic polymer for a polymer-based carrier vehicle for delivery of nucleic acid material to target cells in biological systems, the polymer comprising:
a poly 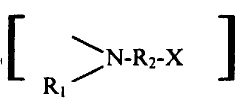 derivative of an amino acid or a synthetic amino acid.
